# EUROPEAN PATENT SPECIFICATION

(11) **EP 2 968 418 B1**
(45) Date of publication and mention of the grant of the patent: **29.11.2023**
(21) Application number: 14762734.3
(22) Date of filing: 14.03.2014
(51) Int. Cl.: A61L 27/36, A61L 27/38, A61K 35/50, A61K 9/16, A61P 25/00, C12N 5/073

(54) **PREPARATIONS DERIVED FROM PLACENTAL MATERIALS OF MAKING AND USING SAME**
AUS PLAZENTAMATERIAL GEWONNENE PRÄPARATE SOWIE VERFAHREN ZUR HERSTELLUNG UND VERWENDUNG DAVON
PRÉPARATIONS DÉRIVÉES DE MATÉRIAUX PLACENTAIRES ET FABRICATION ET UTILISATION DE CELLES-CI

(30) Priority: 15.03.2013 US 201361789785 P
(43) Date of publication of application: 20.01.2016
(60) Divisional application: 23196259.8
(73) Proprietor: Nutech Medical, Inc., Birmingham, AL 35216 (US)
(72) Inventor: VINES, Jeremy, B., Birmingham, AL 35216 (US); WALTHALL, Howard, P., Jr., Birmingham, AL 35213 (US)
(74) Representative: Elsy, David
(86) International application number: PCT/US2014/028207
(87) International publication number: WO 2014/143990

(56) References cited:
- EP-A1- 1 845 154
- WO-A2-2007/079183
- WO-A2-2010/026574
- US-A1- 2008 299 087
- ALVIANO F ET AL: "Term amniotic membrane is a high throughput source for multipotent mesenchymal stem cells with the ability to differentiate into endothelial cells in vitro", BMC DEVELOPMENTAL BIOLOGY, BIOMED CENTRAL LTD., LONDON, GB, vol. 7, no. 1, 21 February 2007 (2007-02-21), page 11, XP021021998, ISSN: 1471-213X, DOI: 10.1186/1471-213X-7-11
- SEMENOV O V ET AL: "Multipotent mesenchymal stem cells from human placenta: critical parameters for isolation and maintenance of stemness after isolation", AMERICAN JOURNAL OF OBSTETRICS & GYNECOLOGY, MOSBY, ST LOUIS, MO, US, vol. 202, no. 2, 1 February 2010 (2010-02-01), pages 193.e1-193.e13, XP026869557, ISSN: 0002-9378 [retrieved on 2010-01-26]
- ALVIANO, F. ET AL.: 'Term amniotic membrane is a high throughput source for multipotent mesenchymal stem cells with the ability to differentiate into endothelial cells in vitro' BMC DEVELOPMENTAL BIOLOGY vol. 7, no. 11, 2007, XP021021998
- PRESTWICH, G. D.: 'Hyaluronic acid-based clinical biomaterials derived for cell and molecule delivery in regenerative medicine' JOURNAL OF CONTROLLED RELEASE vol. 155, no. 2, 2011, pages 193 - 199, XP055011774
- BACENKOV, D. ET AL.: 'Isolation and basic characterization of human term amnion and chorion mesenchymal stromal cells' CYTOTHERAPY vol. 13, no. 9, 2011, pages 1047 - 1056, XP055291213
- NAZAROV, I. ET AL.: 'Multipotent stromal stem cells from human placenta demonstrate high therapeutic potential' STEM CELLS TRANSLATIONAL MEDICINE vol. 1, no. 5, 2012, pages 359 - 372, XP055283323
- SEMENOV, 0. V. ET AL.: 'Multipotent mesenchymal stem cells from human placenta: critical parameters for isolation and maintenance of stemness after isolation' AMERICAN JOURNAL OF OBSTETRICS AND GYNECOLOGY vol. 202, no. 2, 2010, pages 193.EL - 193.E13, XP026869557

## Description

### Related Applications

This application claims priority to U.S. Provisional Patent Application No. 61/789,785, filed on March 15, 2013, and titled, "Preparations Derived from Placental Materials and Methods of Making and Using Same".

### Technical Field

The present invention is directed to preparations derived from placental materials and methods of making and using same.

### Background of the Invention

The placenta surrounds a fetus during gestation and is composed of, among other tissues, an inner amniotic layer that faces the fetus and a generally-inelastic outer shell, or chorion. The placenta anchors the fetus to the uterine wall, allowing nutrient uptake, waste elimination, and gas exchange to occur via the mother's blood supply. Additionally, the placenta protects the fetus from an immune response from the mother. From the placenta, an intact placental membrane comprising the amnion and chorion layers can be separated from the other tissues. The amnion and chorion layers may be used together, or physically separated and used individually.

Clinicians have used intact placental membrane, comprising an amnion and a chorion layer, in medical procedures since as early as 1910 (Davis, J.S., John Hopkins Med. J. 15, 307 (1910)). The amniotic membrane, when separated from the intact placental membrane, may also be used for its beneficial clinical properties (Niknejad H, et al. Eur Cell Mater 15, 88-99 (2008)). Certain characteristics of the placental membrane make it attractive for use by the medical community. These characteristics include, but are not limited to, its anti-adhesive, anti-microbial, and anti-inflammatory properties; wound protection; ability to induce epithelialization; and pain reduction. (Mermet I, et al. Wound Repair and Regeneration, 15:459 (2007)).

Other uses for placental membrane include its use for scaffolding or providing structure for the regrowth of cells and tissue. An important advantage of placental membrane in scaffolding is that the amnion contains an epithelial layer. The epithelial cells derived from this layer are similar to stem cells, allowing the cells to differentiate into cells of the type that surrounds them. Pluripotent cells similar to stem cells are also contained within the body of the amniotic membrane. Additionally, the amniotic membrane contains various growth and trophic factors, such as epidermal, insulin-like, and fibroblast growth factors, as well as high concentrations of hyaluronic acid, that may be beneficial to prevent scarring and inflammation and to support healing. Thus, placental membrane offers a wide variety of beneficial medical uses.

EP1845154 A1 describes a method of providing placenta-derived multipotent stem cells, a production method of placenta-derived multipotent stem cells, and a method for differentiating placenta-derived multipotent stem cells.

Cell-based therapies have considerable potential for the repair and regeneration of tissues. The addition of a matrix or scaffold to these cell-based therapies has yielded improved outcomes (Krishnamurithy G, et al. J Biomed Mater Res Part A 99A, 500-506 (2011)). Ideally, the material used will be biocompatible such that it provokes little to no immune response, biodegrades, and is available in sufficient quantities to be practical. Although the placental membrane has long been identified as a materially potentially filling this role in the clinic, efforts have been limited to *in vitro* studies, impractical *in vivo* techniques, or have yielded less than optimal outcomes. Furthermore, the conditions under which the scaffold is used may have a dramatic effect on the therapeutic efficacy.

While a number of placental membrane products have been studied in the literature or used clinically, these so far fall into two primary categories. The first category involves the use of the intact membrane, be it fresh, dried, freeze-dried, cryopreserved, or preserved in glycerol or alcohol. In this formulation, the membrane is useful for a number of purposes, but is not suitable for others, such as applications requiring injection, or the filling of a space which does not conform to the thin planar shape of the membrane itself.

The second category involves the grinding, pulverizing and/or homogenizing of the membrane into small particles, which may then be resuspended in solution. Such techniques are described, for example, in US2008/299087 (U.S. Patent App. No. 11/528,902); US2007/071740 (U.S. Patent App. No. 11/528,980); US2007/231401 (U.S. Patent App. No. 11/529,658); and US2007/071828 (U.S. Patent App. No. 11/535,924). This grinding may be done dry or wet, and temperature during grinding may or may not be controlled, such as in the case of cryogrinding. Products produced using this method are useful for a number of applications, and may be injected under appropriate conditions. However, they have several deficiencies for certain applications. First, the cells contained in the placental membranes will be destroyed during the grinding process. Second, proteins and growth factors in the membrane may be leached out or lost during this process, including any subsequent washing or other treatment of the ground particles. Indeed, the removal of potentially angiogenic factors such as growth factors may be an objective of this type of processing. Third, resuspension of these small particles in typical physiologic solutions, such as saline, results in a free-flowing fluid with low viscosity. Upon injection or placement, this fluid may dissipate rather than remain in the desired treatment location.

Therefore a need remains for preparations which will allow the delivery of the benefits of the placental membranes across a variety of surgical applications.

### Summary of the Invention

The present invention is defined in the appended claims, and is directed to preparations derived from placental materials and methods of making and using same. The invention is directed to a method of making a preparation comprising the steps of deriving multipotent cells from digested placental membranes; and growing the multipotent cells in adherent culture beyond confluence to form a preparation; wherein the preparation comprises hyaluronic acid, collagen, proteins, and growth factors.

In one embodiment, placental membranes such as the amnion, the chorion, or both membranes together are digested using collagenase, with or without pre-treatment by trypsin. This results in the digestion of the collagen structure of the membrane, leaving behind a viscous, gel-like substance containing the remaining components of the membrane, including cells, proteins, and hyaluronic acid. Processing is carried out so as to preserve, to the extent possible, the protein content of the membrane, including growth factors. This material may then be injected, sprayed or placed into a surgical site.

It has surprisingly been discovered that multipotent cells cultured from the placental membranes and grown in adherent culture beyond confluence produce a unique biomaterial, believed to be composed primarily of HA and collagen, and incorporating growth factors and other proteins. Such material may be used, with or without removal of the cells, in a minced or solid form for tissue repair applications, or may be prepared as an injectable by grinding in accordance with the established techniques in the art, or via collagenase digestion as disclosed herein.

Injectable preparations as disclosed herein can be used for a variety of cosmetic and/or plastic surgery applications, including nerve repair, bony healing, as a dermal filler for dermatology and plastic surgery applications, treatment of damaged muscle or myocardial tissue, and for enhancement of tissue repair methods such as Anterior Cruciate Ligament (ACL) repair and Rotator Cuff Repair.

In one embodiment, the method of making a preparation may further comprise the step of adding to the preparation a biocompatible material selected from the group consisting of collagen, fibrin, silk proteins, keratin and combinations thereof. In another embodiment, the preparation may include cells isolated from amniotic fluid or placental tissue.

In another embodiment, the placental membranes may be ground using techniques known in the art, and the resulting particles resuspended in a fluid containing hyaluronic acid. Such processing should be carried out so as to preserve, to the extent possible, the protein content of the membrane, including growth factors. Preferably, grinding should be conducted under temperature controlled conditions, such as in a cryomill. Hyaluronic acid (HA), or hyaluronan, is a linear polysaccharide that consists of alternating units of a repeating disaccharide, ß -1,4- D -glucuronic acid- ß - 1,3- N -acetyl- D -glucosamine. HA is found throughout the body, from the vitreous of the eye to the extracellular matrix (ECM) of various tissues. HA is an essential component of the ECM, believed to be involved in cellular signaling, wound repair, morphogenesis, and matrix organization. Additionally, HA is rapidly turned over in the body by hyaluronidase, with half-lives ranging from hours to days. HA and its derivatives have been used clinically in a variety of applications. For example, HA in an injectable form is used routinely in ocular surgery, as a dermal filler, and in the treatment of arthritis of the synovial joints. Depending on the molecular weight of the HA selected, the resulting preparation is injectable but viscous. This injectable product may then be used to treat degeneration of the spinal disc by encouraging the regeneration of the disc and the restoration of disc height.

In one embodiment, the method of making a preparation may also comprise a step of lysing cells in the preparation using a process selected from the group consisting of irradiating the preparation, soaking the preparation in a hypertonic solution or both.

A further understanding of the nature and advantages of the present invention will be realized by reference to the remaining portions of the specification.

### Detailed Description of the Invention

### A. Placental Membrane Preparations and Methods of Making Same

### 1. Initial Treatment and Removal of Particular Layers of the Placental Membrane.

Placental membrane sheets may be produced from placentas collected from consenting donors in accordance with the Current Good Tissue Practice guidelines promulgated by the U.S. Food and Drug Administration.

In particular, soon after the birth of a human infant via a Cesarean section delivery, the intact placenta is retrieved, and the placental membrane is dissected from the placenta. Afterwards, the placental membrane is cleaned of residual blood, placed in a bath of sterile solution, stored on ice and shipped for processing. Once received by the processor, the placental membrane is rinsed to remove any remaining blood clots, and if desired, rinsed further in an antibiotic rinse (Diaz-Prado SM, et al. Cell Tissue Bank 11, 183-195 (2010)).

The antibiotic rinse may include, but is not limited to, the antibiotics: amikacin, aminoglycosides, amoxicillin, ampicillin, ansamycins, arsphenamine, azithromycin, azlocillin, aztreonam, bacitracin, capreomycin, carbacephem, carbapenems, carbenicillin, cefaclor, cefadroxil, cefalexin, cefalotin, cefamandole, cefazolin, cefdinir, cefditoren, cefepime, cefixime, cefoperazone, cefotaxime, cefoxitin, cefpodoxime, cefprozil, ceftaroline fosamil, ceftazidime, ceftibuten, ceftizoxime, ceftobiprole, ceftriaxone, cefuroxime, chloramphenicol, ciprofloxacin, clarithromycin, clindamycin, clofazimine, cioxacillin, colistin, cycloserine, dapsone, daptomycin, demeclocycline, dicloxacillin, dirithromycin, doripenem, doxycycline, enoxacin, ertapenem, erythromycin, ethambutol, ethionamide, flucioxacillin, fosfomycin, furazolidone, fusidic acid, gatifloxacin, geldanamycin, gentamicin, glycopeptides, grepafloxacin, herbimycin, imipenem or cilastatin, isoniazid, kanamycin, levofloxacin, lincomycin, lincosamides, linezolid, lipopeptide, lomefloxacin, loracarbef, macrolides, mafenide, meropenem, methicillin, metronidazole, mezlocillin, minocycline, monobactams, moxifloxacin, mupirocin, nafcillin, nalidixic acid, neomycin, netilmicin, nitrofurans, nitrofurantoin, norfloxacin, ofloxacin, oxacillin, oxytetracycline, paromomycin, penicillin G, penicillin V, piperacillin, platensimycin, polymyxin B, pyrazinamide, quinolones, quinupristin/dalfopristin, rifabutin, rifampicin or rifampin, rifapentine, rifaximin, roxithromycin, silver sulfadiazine, Sparfloxacin, spectinomycin, spiramycin, streptomycin, sulfacetamide, sulfadiazine, sulfamethizole, sulfamethoxazole, sulfanilamide, sulfasalazine, sulfisoxazole, sulfonamidochrysoidine, teicoplanin, telavancin, telithromycin, temafloxacin, temocillin, tetracycline, thiamphenicol, ticarcillin, tigecycline, tinidazole, tobramycin, trimethoprim, trimethoprimsulfamethoxazole (co-trimoxazole) (TMP-SMX), and troleandomycin, trovafloxacin, or vancomycin.

The antibiotic rinse may also include, but is not limited to, the antimycotics: abafungin, albaconazole, amorolfin, amphotericin B, anidulafungin, bifonazole, butenafine, butoconazole, caspofungin, clotrimazole, econazole, fenticonazole, fluconazole, isavuconazole, isoconazole, itraconazole, ketoconazole, micafungin, miconazole, naftifine, nystatin, omoconazole, oxiconazole, posaconazole, ravuconazole, sertaconazole, sulconazole, terbinafine, terconazole, tioconazole, voriconazole, or other agents or compounds with one or more anti-fungal characteristics.

The placental membrane may be processed to remove one or more particular layers of the membrane. The chorion may be removed from the placental membrane by mechanical means well-known to those skilled in the art. The chorion may be removed, for example, by carefully peeling the chorion from the remainder of the placental membrane using blunt dissection (Jin CZ, et al. Tiss Eng 13, 693-702 (2007)). Removal of the epithelial layer from the placental membrane may be achieved using several methods well-known to those skilled in the art. The epithelial layer may be preserved or, if desired, may be removed by, for example, using trypsin to induce necrosis in the epithelial cells (Diaz-Prado SM, et al. Cell Tissue Bank 11, 183-195 (2010)). Removal of the epithelial layer may comprise, for example, treatment with 0.1% trypsinethylenediaminetetraacetic acid (EDTA) solution at 37°C for 15 minutes followed by physical removal using a cell scraper (Jin CZ, et al. Tiss Eng 13, 693-702 (2007)).

### 2. Collagenase Digestion of Placental Membranes.

In one embodiment, a placental membrane is digested in collagenase. The collagenase chosen may be one or more of a variety of molecules with collagen-digestion activity known in the art. The membrane selected may be the amnion, the chorion, or both together. The epithelial layer may be permitted to remain in place for digestion, or may be removed using trypsin treatment or other techniques known in the art. The membrane may be cut into strips or pieces, or minced prior to collagenase treatment. The application of collagenase results in the digestion of the collagen structure of the membrane, leaving behind a viscous, gel-like substance containing the remaining components of the membrane, including cells, proteins, and hyaluronic acid. The cells living after collagenase treatment may be preserved. Alternatively, using techniques known in the art, the cells may be lysed before or after collagenase treatment using, for example, irradiation, or the soaking of the membrane in a hypertonic solution. Processing may be carried out so as to preserve, to the extent possible, the protein content of the membrane, including growth factors.

The resulting material may be cryopreserved by freezing, with or without the addition of other multipotent cells, such as cells from the amniotic fluid. Alternatively, the material may be dried or freeze-dried for storage, and reconstituted using a physiologic solution. For example, sterile isotonic saline, or a sterile buffered saline solution (e.g. U.S. Patent Application Publication No. 2003-0187515), may be used. The material may also be stored in solution at various temperatures, using techniques known in the art. The material may be combined with other biocompatible materials, such as collagen, hyaluronic acid, fibrin, gelatin, or various pharmacologic compounds. If preservation of living cells is not desired, the material may be sterilized, typically using irradiation, as is well-known to those skilled in the art. Approximately 25 kGy gamma irradiation, for example, may be used for sterilization (Krishnamurithy G., et al. J Biomed Mater Res Part A 99A, 500-506 (2011)). The material, with or without the addition of other biocompatible materials or solvents, may be injected, sprayed or placed into a surgical site.

By way of example only, the following techniques may be used:
1. Wash membrane 3 times in PBS w/o Ca++ and Mg++ in a sterile fluid basin
2. Place membrane into 0.25% trypsin w/EDTA and incubate @ 37°C in cell culture incubator for 15 mins in 50 cc tube. Use 8mL for every 16 cm² of membrane.
3. Take out membrane and wash 3 times in PBS w/o Ca++ and Mg++ in a sterile fluid basin
4. Place membrane into 8mL of collagenase type II (2mg/mL type II in DMEM w/Hepes buffer) solution for every 16cm² section of membrane and mince membrane into pieces w/ scissors. Place in incubator and incubate @ 37 °C for 3 hours
5. Take membrane out of incubator, pipette membrane up and down vigorously and run through 100um cell strainer mesh into 50cc Tube.
6. Centrifuge @ 1000 rpm for 5 minutes
7. Aspirate supernatant
8. Wash pellet with PBS
9. Centrifuged @ 1000rpm for 5 minutes
10. Aspirate Supernatant

The material remaining behind after aspiration may then be used, or further stored or processed as disclosed herein.

### 3. Preparations of Hyaluronic Acid and Collagen Derived from Digested Placental Membranes.

It has surprisingly been discovered that multipotent cells derived from digested placental membranes and grown in adherent culture beyond confluence produce a unique biomaterial, believed to be composed primarily of HA and collagen, and incorporating growth factors and other proteins. Such discovery was surprising because cells grown in culture typically exhibit contact inhibition after reaching confluence, and do not typically continue to grow or produce a matrix-like material. The adherent culture may be grown in a culture flask or using other appropriate culture arrangements, well known in the art. The resulting material may be used, with or without removal of the cells, in a minced or solid form for tissue repair applications, or may be prepared as an injectable by grinding in accordance with the established techniques in the art, or via collagenase digestion as disclosed herein.

### 4. Placental Membrane Particles Suspended in HA.

In another embodiment, the placental membranes may be ground using techniques known in the art, and the resulting particles re-suspended in a fluid containing hyaluronic acid. Such processing may be carried out so as to preserve, to the extent possible, the protein content of the membrane, including growth factors. Preferably, grinding should be conducted under temperature controlled conditions, such as in a cryomill. As is well known in the art, the HA selected may be of various molecular weights. It may be derived from various human or animal sources, or may be produced in a recombinant fashion, such as by genetically modified bacteria. Depending on the molecular weight of the HA selected, the resulting preparation is injectable but viscous. This injectable material may then be used to treat degeneration of the spinal disc. The injectable material may be combined with other biocompatible materials, such as, for example, collagen, fibrin, silk proteins, or keratin. The injectable may be combined with, or followed by, the injection of a fibrin sealant or other adhesive matrix to seal the hole in the disc created by the injection.

### B. Uses for the Placental Membrane Preparations

The embodiments of the preparation, described herein, may be used to regenerate damaged or defective tissue.

### 1. Connective Tissue Repair.

Due to trauma, overuse, and for other reasons, ligaments, tendons and other connective tissues in the body may fully or partially tear, requiring surgical repair. Common examples include repair of the cruciate and medial collateral ligaments in the knee, the Achilles tendon, and the rotator cuff of the shoulder and hip. While numerous surgical techniques are available for conducting these repairs, in many cases the repairs fail to heal as rapidly and as fully as desired. The healing of rotator cuff repairs is particularly challenging. Additionally, even in the absence of large tears, tendons, ligaments and other connective tissues may become damaged at the microscopic level, resulting in painful and debilitating conditions. The disclosed embodiments may be effectively used in the treatment of such conditions.

The injectable embodiments may be used to treat tendon, ligament and connective tissue injuries by direct injection of the affected area. Similarly, one or more injections into the joint capsule or at the repair site following a reparative surgery of the connective tissues, such as, for example, ACL repair or Rotator Cuff repair, should be beneficial in speeding graft incorporation and healing. Alternatively, the repair site or grafting material being used in the surgery, which may be autograft, allograft, xenograft, or synthetic in origin, may be injected with the injectable embodiments prior to or during the surgical procedure.

### 2. Peripheral Nerve Repair.

The management of trauma-associated nerve defects is difficult. While nerve autograft is the gold standard, there are limited sources of motor and sensory nerves and grafting inevitably results in a nerve deficit at the donor site. Prior research has shown that damaged nerves can be surgically repaired using a tubular conduit crossing the defect, and processed allograft nerves have also been successfully used for this purpose. However, healing is slow and particularly for larger nerve gaps often results in less than satisfactory recovery of function. Accordingly it has been suggested that the inclusion of an appropriate biomaterial within the tubular conduit may improve functional outcomes. (Sierpinski, Paulina, et. al., The Use of Keratin Biomaterials Derived from Human Hair for the Promotion of Rapid Regeneration of Peripheral Nerves. Biomaterials 29 (2008) 118-128). Similarly, the addition of neurotrophic factors to a processed human or animal nerve may enhance the performance of the nerve graft. Amniotic materials have been shown to promote nerve growth (Schroeder, Alice, et. al., Effects of the Human Amniotic Membrane on Axonal Outgrowth of Dorsal Root Ganglia Neurons in Culture. Current Eye Research, (2007) 32:731-738). The disclosed embodiments may be effectively used for such purposes.

Specifically, in surgery for which a conduit is used, an injectable embodiment may be incorporated to fill the conduit during the repair surgery. The conduit itself, or a highly porous filler material, may be soaked in an injectable embodiment. Alternatively, a solid or minced form of the biomaterial produced by over-confluent placental cells in culture may be used as a filler for the conduit. In surgery for which a processed nerve is to be used, the nerve may itself be injected with an injectable embodiment.

### 3. Wound and Burn Care.

The treatment of large-scale bums and non-healing wounds is extremely challenging. Though many treatment options are available, some wounds are recalcitrant to treatment, and many do not heal with satisfactory results. Since at least 1910, amniotic membranes have been used in the treatment of bums and chronic wounds. Accordingly, it is believed that the disclosed invention may be beneficially used in the treatment of non-healing wounds and bums. Specifically, an injectable embodiment may be injected around or in the wound bed, stimulating healing.

### 4. Bone Growth and Fusion.

Products incorporating ground amniotic membrane, commercially including NuCel^{®} (an allograft derived from human amnion and amniotic fluid, available from NuTech Medical Inc. of Birmingham, Alabama), BioDFactor^{™} (a cryopreserved injectable allograft derived from human placental tissues, available from BioD, LLC of Memphis, Tennessee) and Ovation^{®} (a cellular repair matrix derived from placental mesenchyme, available from Matrix Biosurgical, Inc. of Hermosa Beach, California), have been used with clinical success to promote bone growth and fusion, such as in interbody fusion of the spine. Such products must typically be combined with a hydrophilic matrix prior to use, as they are insufficiently viscous to remain in place without the use of a matrix. In some surgical applications requiring the promotion of bone growth or fusion, however, increased viscosity is desirable, as the placement of adequate matrix or carrier to absorb a liquid product may not be possible or desirable. An injectable embodiment as described herein may be appropriately used as an alternative in such applications.

### 5. Treatment of Skeletal Muscle and Cardiac Muscle.

A variety of factors, including trauma, ischemia, and various disease states, may cause damage to human muscle tissue or otherwise affect the proper functioning of the muscles. Of particular note, cardiac muscle is highly susceptible to ischemic damage from blockage of the cardiac vasculature. Amniotic products and cells have been shown to be beneficial for muscle repair and recovery. Accordingly, the disclosed embodiments could be used, alone or in combination with other materials, in the treatment of injuries to or diseases of the skeletal and cardiac musculature.

### 6. Dermal Filler.

There are numerous cosmetic and plastic surgery applications in which dermal fillers may be used to restore the appearance of the skin. It is desirable that the products used be injectable, but sufficiently viscous to remain in place after injection. Numerous products have been investigated or used clinically. (Kontis, Theda C., Contemporary Review of Injectable Facial Fillers. Facial Plast Surg. 2013; 15(1): 5864). Amniotic materials have been shown to support the repair and healing of the skin and connective tissue. The described embodiments, with or without the addition of other materials, such as, for example, hyaluronic acid, may be appropriately used as an injectable dermal filler.

### 7. Spinal Disc Degeneration.

Intervertebral discs are fibrocartilaginous tissues occupying the space between vertebral bodies in the spine. They transmit forces from one vertebra to the next, while allowing spinal mobility. The structural properties of the disc are largely depending on its ability to attract and retain water. Proteoglycans in the disc exert an osmotic "swelling pressure" that resists compressive loads. Degeneration of the intervertebral disc is a physiologic process that is characteristic of aging in humans. With age, the disc undergoes a variety of changes, the most notable being a loss of proteoglycan content resulting in reduced osmotic pressure and a reduction in disc height and ability to transmit loads. Disc degeneration is an important and direct cause of spinal conditions that account for most neck and back pain.

As is the case with the related cartilage and tendon cells, components of the amniotic membrane may promote healing and recovery of the intervertebral disc and associated cells. It is believed that the disclosed invention may be beneficially used in the treatment of degenerative disc disease. Specifically, an injectable embodiment may be injected into the disc. The embodiment may or may not contain living cells from the placental membrane. Additional cells isolated from the amniotic fluid of the same donor using techniques known in the art may or may not be added. The injectable material may be combined with other biocompatible materials, such as, for example, hyaluronic acid, collagen, fibrin, silk proteins, or keratin. The injectable may be combined with, or followed by, the injection of a fibrin sealant or other adhesive matrix (Buser Z, et. al., Biological and Biomechanical Effects of Fibrin Injection into Porcine Intervertebral Discs. Spine (2011) 36(18)1201-1209) to seal the hole in the disc created by the injection.

## Claims

1. A method of making a preparation comprising the steps of
deriving multipotent cells from digested placental membranes;
growing the multipotent cells in adherent culture beyond confluence to form a preparation;
wherein the preparation comprises hyaluronic acid, collagen, proteins, and growth factors.

2. The method according to claim 1 wherein the placental membrane includes the amnion and chorion together.

3. The method according to claim 1 wherein the preparation is minced.

4. The method according to claim 1, comprising the additional step of grinding the preparation in a manner suitable for injection.

5. The method according to claim 4 comprising the additional step of re-suspending the ground preparation in a fluid containing hyaluronic acid.

6. The method according to claim 5 wherein the grinding is conducted in a cryomill.

7. The method according to claim 1 wherein the preparation is digested via collagenase.

8. The method according to any one of claims 5 to 7 further comprising the step of adding to the preparation a biocompatible material selected from the group consisting of collagen, fibrin, silk proteins, keratin and combinations thereof.

9. The method according to any one of claims 1 to 8 wherein the preparation includes cells isolated from amniotic fluid or placental tissue.

10. The placental membrane preparation obtained by the method of any one of claims 1 to 9 for cosmetic and/or plastic surgery applications as a dermal filler.

11. The method according to claims 1-9. further comprising the step of lysing cells in the preparation using a process selected from the group consisting of irradiating the preparation, soaking the preparation in a hypertonic solution or both.

## Patentansprüche

1. Verfahren zur Herstellung eines Präparats, umfassend die Schritte:
Ableiten multipotenter Zellen aus verdauten Plazentamembranen;
Züchten multipotenter Zellen in adhärenter Kultur bis zur Konfluenz, um ein Präparat zu bilden;
wobei das Präparat Hyaluronsäure, Kollagen, Proteine und Wachstumsfaktoren umfasst.

2. Verfahren nach Anspruch 1, wobei die Plazentamembran das Amnion und das Chorion zusammen umfassen.

3. Verfahren nach Anspruch 1, wobei das Präparat zerkleinert ist.

4. Verfahren nach Anspruch 1, das den zusätzlichen Schritt des Mahlens des Präparats in einer für die Injektion geeignete Weise umfasst.

5. Verfahren nach Anspruch 4, das den zusätzlichen Schritt des Re-Suspendierens des gemahlenen Präparats in einer Hyaluronsäure enthaltenden Flüssigkeit umfasst.

6. Verfahren nach Anspruch 5, wobei das Mahlen in einer Kryomühle (cryomill) durchgeführt wird.

7. Verfahren nach Anspruch 1, wobei das Präparat durch Kollagenase verdaut wird.

8. Verfahren nach einem der Ansprüche 5 bis 7, das des Weiteren den Schritt des Hinzufügens eines biokompatiblen Materials zu dem Präparat umfasst, wobei das biokompatible Material ausgewählt ist aus der Gruppe bestehend aus Kollagen, Fibrin, Seidenproteine, Keratin und Kombinationen davon.

9. Verfahren nach einem der Ansprüche 1 bis 8, wobei das Präparat Zellen umfasst, die aus Amnionflüssigkeit oder Plazentagewebe isoliert wurden.

10. Plazentamembran-Präparat, das durch das Verfahren nach einem der Ansprüche 1 bis 9 erhalten wurde, für kosmetische und/oder plastische Chirurgie-Anwendungen als Dermalfiller.

11. Verfahren nach den Ansprüchen 1 bis 9, das des Weiteren den Schritt des Lysierens von Zellen in dem Präparat unter Verwendung eines Verfahren umfasst, der ausgewählt ist aus der Gruppe bestehend aus Bestrahlen des Präparats, Durchtränken des Präparats in hypertoner Lösung oder beidem.

## Revendications

1. Procédé de production d'une préparation comprenant les étapes de
obtenir des cellules multipotentes à partir de membranes placentaires digérées;
faire croître les cellules multipotentes dans une culture adhérente au-delà de la confluence pour former une préparation;
dans lequel la préparation comprend de l'acide hyaluronique, du collagène, des protéines et des facteurs de croissance.

2. Procédé selon la revendication 1 dans lequel la membrane placentaire inclut l'amnios et le chorion ensemble.

3. Procédé selon la revendication 1 dans lequel la préparation est émincée.

4. Procédé selon la revendication 1, comprenant l'étape supplémentaire de broyage de la préparation d'une manière adaptée à l'injection.

5. Procédé selon la revendication 4 comprenant l'étape supplémentaire de remise en suspension de la préparation broyée dans un liquide contenant de l'acide hyaluronique.

6. Procédé selon la revendication 5 dans lequel le broyage est effectué dans un cryobroyeur.

7. Procédé selon la revendication 1 dans lequel la préparation est digérée par le biais d'une collagénase.

8. Procédé selon l'une quelconque des revendications 5 à 7 comprenant en outre l'étape d'ajout à la préparation d'un matériau biocompatible choisi dans le groupe consistant en le collagène, la fibrine, les protéines de soie, la kératine et leurs combinaisons.

9. Procédé selon l'une quelconque des revendications 1 à 8 dans lequel la préparation inclut des cellules isolées à partir du liquide amniotique ou du tissu placentaire.

10. Préparation de membrane placentaire obtenue par le procédé selon l'une quelconque des revendications 1 à 9 pour des applications de chirurgie esthétique et/ou plastique comme agent de comblement dermique.

11. Procédé selon les revendications 1 à 9, comprenant en outre l'étape de lyse des cellules dans la préparation au moyen d'un procédé choisi dans le groupe consistant en l'irradiation de la préparation, l'immersion de la préparation dans une solution hypertonique ou les deux.
